# EUROPÄISCHE PATENTSCHRIFT

(11) **EP 2 572 750 B1**
(45) Veröffentlichungstag und Bekanntmachung des Hinweises auf die Patenterteilung: **27.02.2019**
(21) Anmeldenummer: 12198721.8
(22) Anmeldetag: 02.11.2007
(51) Int. Cl.: A61M 5/158, A61B 5/15, A61B 5/153, A61M 39/06, A61M 39/12, A61M 25/06, A61M 39/04, A61M 39/22, A61M 5/32, A61M 39/02

(54) **KATHETERVORRICHTUNG MIT INFUSIONSPORT UND VENTILEN**
Catheter device with infusion port and valves
Dispositif de cathéter avec port de perfusion et soupapes

(30) Priorität: 03.11.2006 US 592595; 30.04.2007 DE 202007006190 U
(43) Veröffentlichungstag der Anmeldung: 27.03.2013
(62) Teilanmeldung aus: 07846527.5
(73) Patentinhaber: B. Braun Melsungen AG, 34212 Melsungen (DE)
(72) Erfinder: Woehr, Kevin, 34212 Melsungen (DE)
(74) Vertreter: Kinkeldey, Daniela

(56) Entgegenhaltungen:
- EP-A1- 0 268 480
- EP-A2- 0 875 262
- WO-A1-94/03232
- WO-A1-99/08742
- US-A1- 2001 053 895
- US-A1- 2002 128 604
- US-A1- 2004 210 194
- US-A1- 2006 178 636

## Beschreibung

Die Erfindung betrifft eine Kathetervorrichtung nach dem Oberbegriff des Anspruchs 1.

Eine Kathetervorrichtung dieser Art ist aus EP 497 576 B1 bekannt, wobei ein Kombinationsventil im Katheteransatz eingesetzt ist, das einerseits die Portöffnung verschließt und andererseits das Austreten von Blut oder Injektionsflüssigkeit aus dem Katheteransatz in proximale Richtung verhindert. An beiden Ventilbereichen kann Injektionsflüssigkeit eingeführt werden, indem die jeweiligen Ventilbereiche verformt werden.

US 2004/0210194 A1 beschreibt eine Thrombektomiekatheter-Vorrichtung mit einem selbstabdichtenden Hämostase-Ventil, das derart eingestellt werden kann, dass es unabhängig von der Verwendung eines darin eingeschlossenen Führungsdrahts bei einem gewünschten Druck automatisch abdichtet. Das Ventil 64 hat die Funktion, einen eingeführten Führungsdraht 48 abzudichten und ist selbstabdichtend, wenn kein Führungsdraht vorhanden ist.

US 2006/0178636 A1 offenbart eine Vorrichtung zum selektiven Schließen von Zugängen innerhalb eines Katheters umfassend ein Leitungsrohr 14 mit einem rückseitigen Ende für die Einführung eines medizinischen Instruments und einen Mechanismus 27 für die Justierung des Querschnitts des Leitungsrohrs 14. Ein derartiges Instrument wird insbesondere bei Angioplastie-Verfahren in der Kardiologie verwendet, wodurch Blutgefäße (Venen oder Arterien), die sich verengt haben, geformt und geweitet werden, so dass ein Stent eingesetzt werden kann. Des Weiteren werden Abdichtungen ("seal 60, 64") beschrieben, die mit Hilfe einer Kanüle ("cannula 74") geöffnet werden können. Das Öffnen der Abdichtungen 60, 64 ermöglicht die Insertion von medizinischen Instrumenten.

US 2001/0053895 A1 offenbart eine blutfreie Katheter-Über-Nadel-Vorrichtungen mit einer Septum-Abdichtung innerhalb des Katheteransatzes, wobei die Einführnadel durch die Septum-Abdichtung dringt. Die Septum-Abdichtung hat die Funktion, dass nach dem Entfernen der Nadel aus dem Katheter die Abdichtung wieder verschlossen wird, so dass Durchfluss aus dem Katheter bzw. aus dem Patienten verhindert wird. Zum Herstellen einer Verbindung wie etwa einem IV-Beutel, wird ein männlicher Luer-Adapter verwendet, wobei die Abdichtung gegen ein Röhrchen gedrückt wird, das innerhalb des Ansatzes befestigt ist und mit dem Katheter kommuniziert, wodurch ein permanenter Durchgang ausgebildet wird.

EP o 875 262 A2 betrifft eine Vorrichtung, die die Zug- und Reibungskräfte an einem daran verlängerten Teil, wie etwa einem Katheter, vermindern soll. Die darin beschriebene Vorrichtung umfasst ein Mantelgehäuse 20 und ein "Shuttle"-Gehäuse, wobei das Shuttle-Gehäuse relativ zu dem Mantelgehäuse bewegbar ist. In dem Mantelgehäuse ist ein Ventil 28 aus einem Elastomer mit radialen Schlitzen 60 angeordnet. Die Vorrichtung umfasst des Weiteren ein "Shuttle"-Rohr mit einem proximalen und distalen Ende und einen inneren Durchgang, welches beweglich ist und derart ausgestaltet ist, dass es das Ventil öffnet, wenn es in eine Vorwärts-Position bewegt wird.

US 2002/0128604 A1 beschreibt eine Dauerkatheter-Vorrichtung mit einem hohlen Katheteransatz, einem daran verbundenem Katheter und einem elastischen Ventil, das im Katheteransatz angeordnet ist und einem hohlen Stopfen ("plug"), das verschiebbar im Katheteransatz angeordnet ist. Die Einführung einer Verbindung am proximalen Ende des Katheters führt dazu, dass der Stopfen ("plug") gegen das elastische Ventil gedrückt wird, wodurch das Ventil geöffnet.

WO 94/03232 A1 offenbart proximale kardiovaskuläre Katheter-Adapter umfassend einen langgestreckten Körper mit einem proximalen und distalen Ende, die einen zentralen Durchgang ausbilden, mit jeweils einer Öffnung am distalen und proximalen Ende, wobei die Öffnung am distalen Ende an einen proximalen Teil eines Katheters oder ähnlicher Vorrichtungen zur Verwendung in der koronaren Angioplastie bzw. Angiographie angeschlossen werden kann. Diese Katheter-Adapter sind mit einer Abdichtung versehen, um den Austritt von Flüssigkeiten bzw. Blut zu verhindern. In diesem Zusammenhang wird eine passive Abdichtung ("passive seal 24" oder "gasket 24") beschrieben, welche die proximale Öffnung 18 verschließt und somit den Austritt von Blut aus dem Gehäuse verhindert und gleichzeitig eine Injektion von Kathetern und Führungsdrähten erlaubt.

EP o 268 480 A1 beschreibt eine Vorrichtung für die Entnahme bzw. Infusion von Flüssigkeiten aus einem menschlichen Körper umfassend ein erstes Verbindungsstück zum Anschluss an einen Katheter und ein zweites Verbindungsstück zum Anschluss an eine Flüssigkeitsquelle sowie eine Ventilvorrichtung, die einen Durchfluss von Flüssigkeit erlaubt, wenn die Vorrichtung angeschlossen wird.

Der Erfindung liegt die Aufgabe zugrunde, eine solche Kathetervorrichtung mit Port so auszubilden, dass auch eine Flüssigkeitsentnahme, insbesondere Blutentnahme möglich ist.

Diese Aufgabe wird erfindungsgemäß durch den Anspruch 1 gelöst.

Dadurch, dass das zweite Ventilelement als Zwei-Wege-Ventil derart ausgebildet ist, dass eine Durchströmung in beiden Richtungen möglich ist, kann bei Bedarf Flüssigkeit bzw. Blut am proximalen Ende des Katheteransatzes entnommen werden.

Die Erfindung wird beispielsweise anhand der Zeichnung näher erläutert. Es zeigen
- Fig. 1: einen Längsschnitt durch eine erste Ausführungsform der erfindungsgemäßen Kathetervorrichtung mit Port,
- Fig. 2: einen gegenüber Fig. 1 um 90° versetzten Längsschnitt der ersten Ausführungsform mit einer am proximalen Ende eingesetzten Nadel,
- Fig. 3: eine zweite Ausführungsform der erfindungsgemäßen Kathetervorrichtung in der Schnittdarstellung mit einem Ventilöffnungselement,
- Fig. 4: ein in das proximale Ende des Katheteransatzes der zweiten Ausführungsform gemäß Figur 3 eingeführtes Anschlusselement,
- Fig. 5a - 5e: perspektivische Ansichten von Ausführungsformen des Schlauchelementes,
- Fig. 6: einen Längsschnitt einer erfindungsgemäßen Kathetervorrichtung mit dem in Fig. 5b wiedergegebenen Schlauchelement,
- Fig. 7: eine erfindungsgemäße Kathetervorrichtung mit Nadel und einer ersten Ausführungsform eines Nadelschutzelementes,
- Fig. 8a und 8b: ein Ventilbetätigungselement, und
- Fig. 9-12: weitere Ausführungsformen erfindungsgemäßer Kathetervorrichtungen.

In den Figuren ist mit 1 ein hohler Katheteransatz bezeichnet, dessen distales Ende mit einem Katheter 2 verbunden bzw. verbindbar ist. Am proximalen Ende ist der Katheteransatz 1 mit einer Verbindungseinrichtung, beispielsweise einem Luer-Nocken 3 für den Anschluss eines Infusionsschlauches oder dergleichen versehen. Zwischen proximalem und distalem Ende ist am Katheteransatz 1 seitlich ein Port 4 angebracht, mit dem beispielsweise eine Spritze verbunden werden kann, um eine Infusionsflüssigkeit durch den Katheter 2 dem Patienten zuzuführen. Im Bereich der Öffnung 4a des Ports ist im Katheteransatz 1 ein schlauchförmiges, wenigstens teilweise flexibles bzw. elastisches Element 5 angeordnet, das als ein erstes Ventilelement die Portöffnung 4a geschlossen hält und bei Anliegen von Druck an der Portöffnung 4a sich elastisch so verformt, dass eine Verbindung zwischen Portöffnung 4a und Katheter 2 freigegeben wird. Wenn kein Druck mehr an der Portöffnung anliegt, kehrt das Schlauchelement 5 aufgrund seiner Elastizität wieder in die dargestellte Schließstellung zurück.

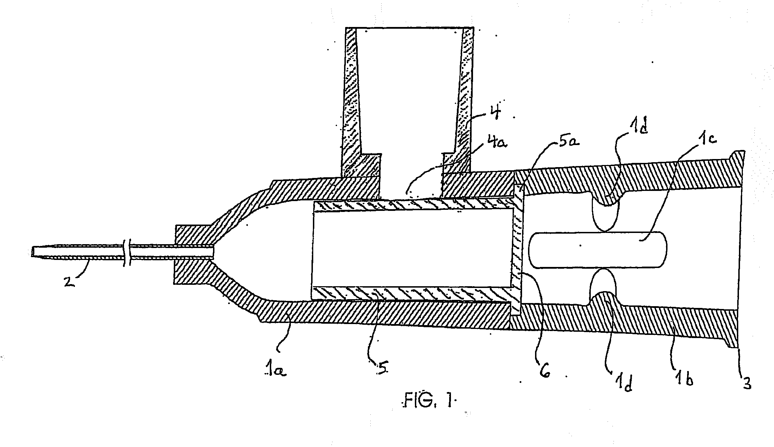

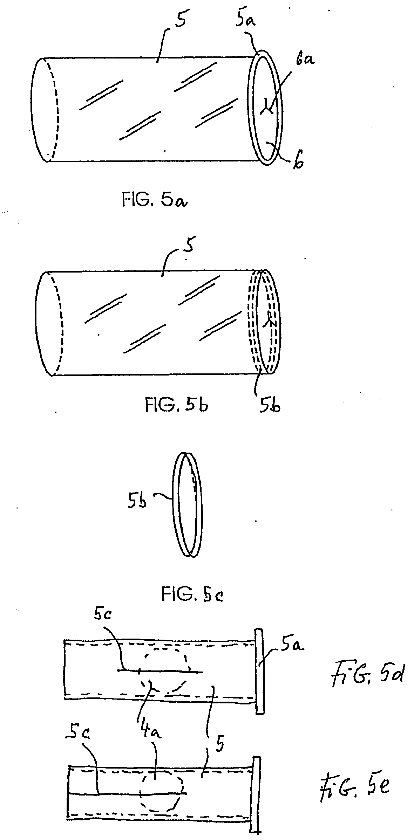

Das wenigstens teilweise flexible Schlauchelement 5 ist bei dem in Fig. 1 wiedergegebenen Ausführungsbeispiel am proximalen Ende durch eine Ventilscheibe 6 verschlossen, die mit wenigstens einem radial verlaufenden Schlitz 6a versehen ist. Fig. 5a zeigt drei sternförmig angeordnete Schlitze 6a. Zweckmäßigerweise besteht die ein zweites Ventil bildende Ventilscheibe 6 aus dem gleichen Material wie das Schlauchelement 5, es kann aber auch ein gesondertes Ventilelement in das proximale Ende oder im proximalen Bereich des Schlauchelementes 5 vor der Portöffnung 4a eingesetzt werden.

Die Ventilscheibe 6 ist als Zwei-Wege-Ventil ausgebildet, sodass eine Durchströmung von der proximalen zur distalen Seite und umgekehrt möglich ist. Damit die Ventilscheibe 6 nicht öffnet, wenn durch den Port 4 Injektionsflüssigkeit eingeführt wird, kann die Ventilscheibe 6 entsprechend steif ausgebildet werden, sodass sie nur für eine Durchströmung in proximale Richtung öffnet, wenn auf der proximalen Seite ein entsprechend starker Saugdruck anliegt, der höher ist als ein am Port 4 angelegter Druck. Vorzugsweise wird ein Ventilbetätigungselement in Verbindung mit der Ventilscheibe 6 vorgesehen, wie dies nachfolgend näher erläutert wird.

Bei dem Ausführungsbeispiel nach Fig. 1 ist am Schlauchelement 5 am proximalen Ende ein radial abstehender Flansch 5a ausgebildet, der zwischen zwei Gehäuseteilen 1a und 1b in einer Nut in Achsrichtung gehalten ist. Durch diesen längs seines Umfangs zwischen den Gehäuseteilen 1a und 1b eingespannten Flansch 5a des Schlauchelementes 5 und die das Schlauchelement am proximalen Ende verschließende und versteifende Ventilscheibe 6 kann sich das Schlauchelement 5 am proximalen Ende nicht radial verformen, wenn an der Portöffnung 4a ein Druck anliegt, sodass das Schlauchelement 5 nur in dem Bereich zwischen distalem Ende und Portöffnung 4a so radial eingedrückt wird, dass eine Verbindung zwischen Portöffnung 4a und Katheter 2 freigegeben wird, während eine Verbindung zwischen Portöffnung 4a und proximalem Ende des Katheteransatzes 1 verschlossen bleibt. Dadurch, dass die Ventilscheibe 6 das proximale Ende des Schlauchelementes 5 abdeckt, kann auch keine Rückströmung der durch den Port 4 eingeführten Infusionsflüssigkeit zum proximalen Ende des Katheteransatzes auftreten.

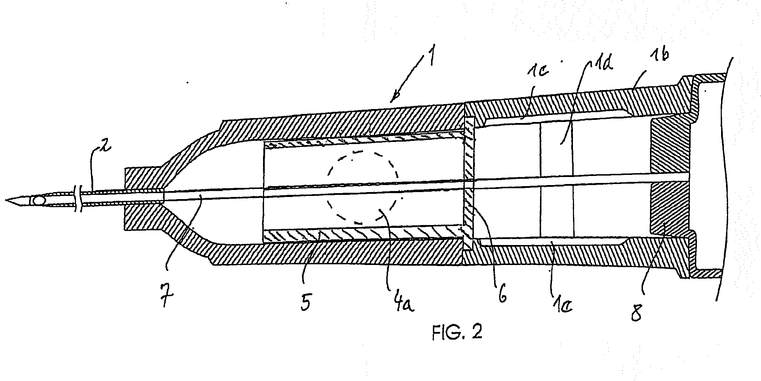

Fig. 2 zeigt eine in die Kathetervorrichtung eingesetzte Hohlnadel 7 an einem Nadelansatz 8, der in das proximale Ende des Katheteransatzes 1 eingesetzt ist, wobei sich die Nadel 7 unter Aufweitung der Schlitze 6a durch die Ventilscheibe 6 und den Katheter 2 erstreckt und mit der Spitze aus dem distalen Ende des Katheters vorsteht.

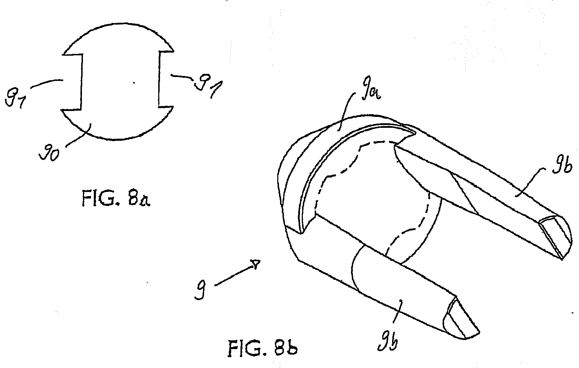

Bei dem Ausführungsbeispiel nach den Fig. 1 und 2 ist im Gehäuseteil 1b auf der proximalen Seite der Ventilscheibe 6 an diametral gegenüberliegenden Stellen eine Längsnut 1c ausgebildet, die zur Führung eines Ventilbetätigungselementes 9 in Achsrichtung des Katheteransatzes 1 dient. Fig. 8b zeigt ein Ausführungsbeispiel eines solchen Ventilbetätigungselementes 9, das auf diametral gegenüberliegenden Seiten eines Kopfteiles 9a zwei Führungsschenkel 9b aufweist, die in Achsrichtung von dem etwa kegelförmigen Kopfteil 9a abstehen und in die Führungsnuten 1c eingreifen.

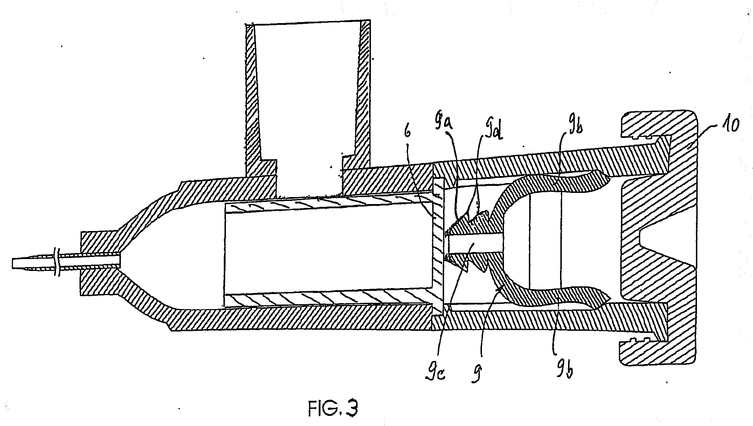

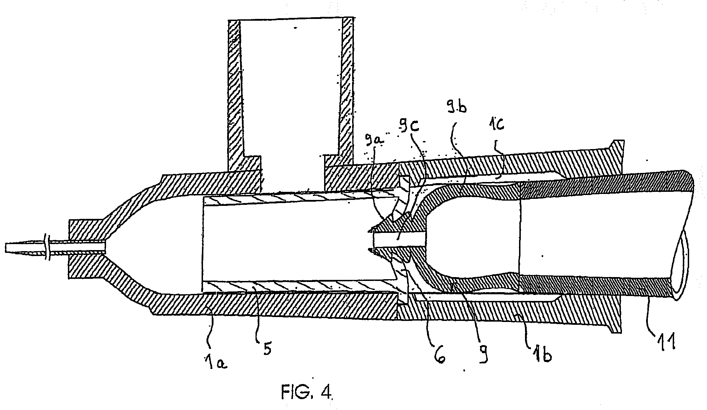

Bei dem Ausführungsbeispiel nach den Fig. 3 und 4 ist das mit einer Durchgangsbohrung 9c versehene Kopfteil 9a mit einer Abstufung 9d versehen, damit das Ventilbetätigungselement 9 in der Ventilscheibe 6 nach Auseinanderdrücken der radialen Schlitze 6a einrasten kann, wie dies Fig. 4 zeigt. Hierbei ist ein rohrförmiges Anschlusselement 11 in das proximale Ende des Katheteransatzes 1 eingeführt, dessen Stirnseite an den Enden der Führungsschenkel 9b zum Anliegen kommt und diese in distale Richtung so verschiebt, dass das Kopfteil 9a des Ventilbetätigungselementes 9 die Ventilscheibe 6 öffnet.

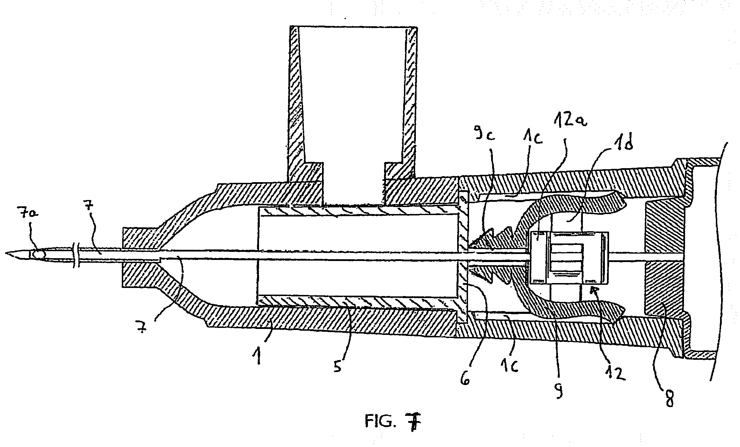

Fig. 7 zeigt die in die Kathetervorrichtung eingesetzte Hohlnadel 7, die sich durch die Bohrung 9c im Ventilbetätigungselement 9 und die Ventilscheibe 6 erstreckt, wobei zwischen Nadelansatz 8 und Ventilbetätigungselement 9 ein Nadelschutzelement 12 auf der Nadel 7 angeordnet ist, das im Katheteransatz 1 in der in Fig. 7 wiedergegebenen Bereitstellung an einem radialen Vorsprung 1d gehalten wird, der sich bei dem dargestellten Ausführungsbeispiel in Umfangsrichtung zwischen den Führungsnuten 1c auf dem Innenumfang des Gehäuseteils 1b des Katheteransatzes erstreckt. Fig. 1 zeigt die beiderseits der Führungsnut 1c ausgebildeten, wulstförmigen Abschnitte des radialen Vorsprungs 1d teilweise im Schnitt. Wenn die Nadel 7 aus der in Fig. 7 wiedergegebenen Bereitstellung des Nadelschutzelementes 12 aus dem Katheteransatz zurückgezogen wird, wird das Nadelschutzelement 12 im Katheteransatz 1 zunächst durch den radialen Vorsprung 1d gehalten, bis eine nahe der Nadelspitze vorgesehene Eingriffseinrichtung 7a vorzugsweise in der Form einer Nadelquetschung, die zu diametral gegenüberliegenden Vorsprüngen am Nadelumfang führt, mit der proximalen Rückwand des Nadelschutzelementes 12 in Eingriff tritt und bei weiterem Zurückziehen der Nadel 7 das Nadelschutzelement 12 mitnimmt, wobei die distalen Enden der Arme 12a die Nadelspitze übergreifen und blockieren, indem sie sich radial nach innen bewegen und sich dabei von dem radialen Vorsprung 1d lösen, sodass das Nadelschutzelement 12 zusammen mit der Nadel aus der Kathetervorrichtung herausgezogen werden kann.

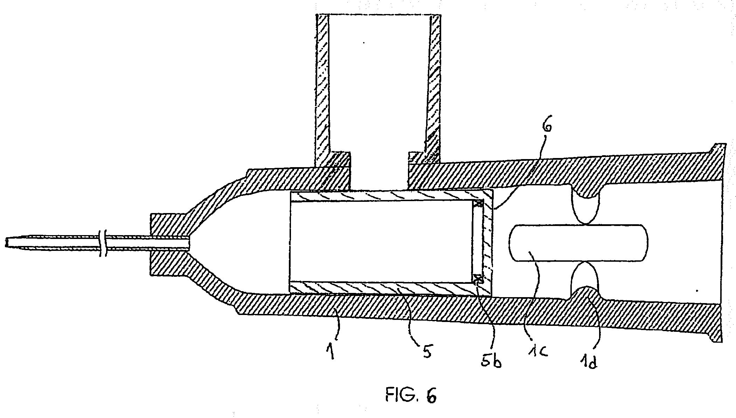

Das Schlauchelement 5 kann am proximalen Ende oder im proximalen Bereich auch durch einen innen liegenden Ring 5b versteift ausgebildet werden, wie dies Fig. 5b und Fig. 5c zeigen. Fig. 6 zeigt einen Längsschnitt durch eine Kathetervorrichtung mit einem am proximalen Ende derart versteiften Schlauchelement 5. Bei dieser Ausführungsform des Schlauchelementes kann der Katheteransatz 1 auch einstückig ausgebildet sein, wie Fig. 6 zeigt. Der nicht flexible Versteifungsring 5b kann auch in einem Abstand vom proximalen Ende des Schlauchelementes in dieses eingesetzt sein, wobei er zwischen proximalem Ende des Schlauchelementes und Portöffnung 4a angeordnet wird..

Ein Versteifungsring 5b oder ein entsprechendes Versteifungselement kann auch in Verbindung mit einem Flansch 5a vorgesehen werden.

Fig. 5d zeigt eine Ausführungsform des Schlauchelementes 5, das einen Schlitz 5c in Achsrichtung im Bereich der Portöffnung 4a aufweist, um bei Einführen von Injektionsflüssigkeit durch den Port 4 ein Öffnen des Schlauchelementes 5 zu erleichtern. Bei der in Fig. 5e wiedergegebenen Ausführungsform erstreckt sich der Schlitz vom Bereich der Portöffnung 4a bis zum distalen Ende des Schlauchelementes 5, während der Schlitz bei der Ausführungsform nach Fig. 5d nur im Bereich der Portöffnung ausgebildet ist, sodass beim Einführen von Injektionsflüssigkeit durch den Port 4 sich nur die Ränder des Schlitzes verformen und der distale Abschnitt des Schlauchelementes am Innenumfang des Katheteransatzes anliegend bleiben kann.

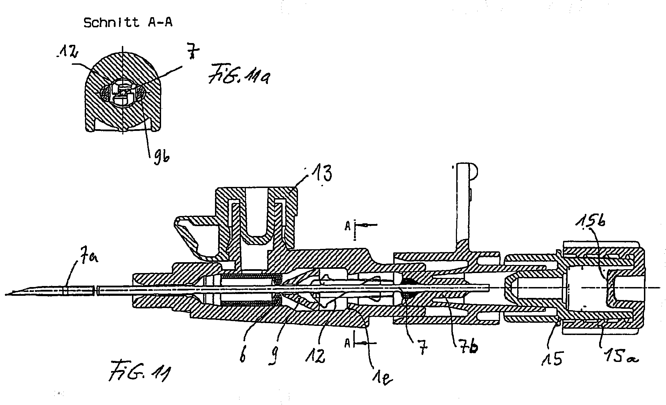

Das proximale Ende des Katheteransatzes 1 kann durch eine Verschlusskappe 15a (Fig. 11) verschlossen gehalten werden, wenn über den Port 4 eine Injektion ausgeführt wird.

Anstelle der beschriebenen Ausführungsform eines flexiblen Schlauchelementes 5, das sowohl ein erstes Ventilelement an der Portöffnung 4a als auch ein zweites Ventilelement im proximalen Bereich des Katheteransatzes 1 bildet, können auch für die Portöffnung und die Katheteransatzöffnung jeweils getrennte Ventilelemente vorgesehen sein.

Anstelle eines Ports 4 kann auch eine Kathetervorrichtung mit zwei Ports am Katheteransatz vorgesehen sein, wobei das erste Ventil beide Portöffnungen verschließt und freigibt. Es können dabei auch zwei getrennte Ventilelemente für die beiden Portöffnungen vorgesehen werden.

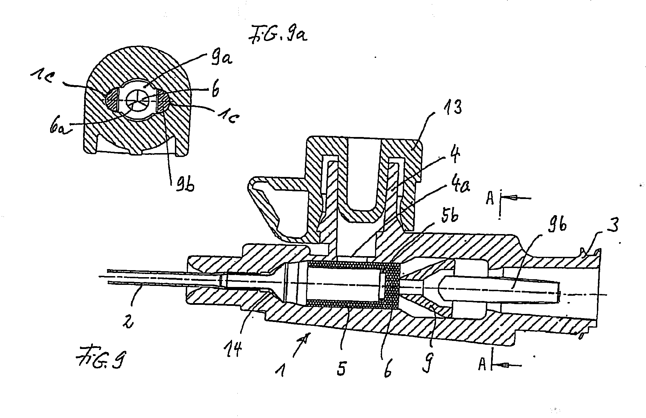

Fig. 9-12 zeigen Ausführungsformen der Kathetervorrichtung, die teilweise denen der Fig. 1-8 entsprechen, wobei für gleiche oder entsprechende Bauteile die gleichen Bezugszeichen verwendet sind.

Fig. 9 zeigt einen einteiligen Katheteransatz 1, bei dem der Port 4 durch eine Kappe 13 verschlossen ist. Der Katheter 2 ist durch ein trichterförmiges Befestigungselement 14 im Katheteransatz 1 durch Klemmen befestigt. Die Wandstärke der Ventilscheibe 6 ist verstärkt gegenüber der Wandstärke am Schlauchelement 5 dargestellt. Hierdurch kann das Schlauchelement 5 bei einem an der Portöffnung 4a anliegenden Druck durch Verformen die Portöffnung 4a öffnen, während die Ventilscheibe 6 geschlossen bleibt. Bei dem dargestellten Ausführungsbeispiel in Fig. 9 ist zusätzlich ein Ventilbetätigungselement 9 vorgesehen, das die Ventilscheibe 6 auf der proximalen Seite abstützt, wenn auf der distalen Seite ein Druck anliegt, sodass die Ventilscheibe in proximale Richtung nicht öffnet, wenn am Port 4 ein Druck anliegt.

Fig. 9a zeigt einen Schnitt längs der Linie A-A in Fig. 9, wobei die sternförmig angeordneten Schlitze 6a der Ventilscheibe 6 durch die Durchgangsöffnung 9c des Ventilbetätigungselementes 9 sichtbar ist. Die in den in Achsrichtung verlaufenden Führungsnuten 1c eingreifenden Führungsschenkel 9b sind an einem trichterförmigen Ventilbetätigungselement 9 angeformt.

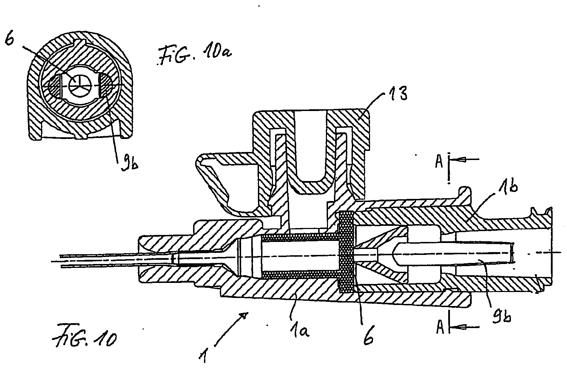

Fig. 10 zeigt in entsprechender Darstellung wie Fig. 9 einen zweiteiligen Katheteransatz 1, bei dem die Ventilscheibe 6 in radialer Richtung verbreitert ausgebildet und durch das Gehäuseteil 1b im Gehäuseteil 1a des Katheteransatzes gehalten ist.

Bei dieser Ausführungsform ist, wie die Schnittansicht in Fig. 10a zeigt, das Gehäuseteil 1b nicht rotationssymmetrisch ausgebildet, sondern im oberen Bereich verstärkt und mit einer in Achsrichtung verlaufenden Rippe auf dem Außenumfang versehen, der in eine entsprechende Längsnut auf dem Innenumfang des Gehäuseteils 1a eingreift. Diese Ausgestaltung verhindert ein Verdrehen zwischen den beiden Gehäuseteilen, wenn z. B. das Anschlusselement 16 angeschraubt wird.

Fig. 11 zeigt eine an einem Nadelansatz 7b befestigte Nadel 7, die sich durch das Ventilbetätigungselement 9, die Ventilscheibe 6 und den Katheter 2 erstreckt, wobei auf der Nadel ein Nadelschutzelement 12 mit sich kreuzenden Armen zwischen den beiden Führungsschenkeln 9b des Ventilbetätigungselementes 9 angeordnet ist. Das Nadelschutzelement 12 in Fig. 11 entspricht dem in Fig. 7 in einer um 90° verdrehten Ansicht Bei der Ausführungsform nach Fig. 11 ist auch der Nadelansatz 7b durch eine Kappe 15 verschlossen. Am proximalen Ende der Kathetervorrichtung ist eine abnehmbare Verschlusskappe 15a aufgesteckt. Nach Entfernen der Nadel 7 mit Nadelschutzelement kann die Verschlusskappe 15a auf das offene proximale Ende des Katheteransatzes aufgeschraubt werden. Vorzugsweise ist die Verschlusskappe 15a mit einem verkürzten Konus 15b versehen, damit das Ventilbetätigungselement 9 durch die Verschlusskappe nicht beaufschlagt wird, wie dies auch in Fig. 3 der Fall ist.

Bei der Ausführungsform nach Fig. 11 wird das Nadelschutzelement 12 im Katheteransatz 1 gehalten, wenn die Nadel 7 aus dem Katheteransatz zurückgezogen wird, indem die gegenüberliegenden Arme an einem Absatz 1e zum Anliegen kommen. Sobald die Nadelspitze zwischen die beiden Arme des Nadelschutzelementes 12 zu liegen kommt, schwenken die beiden Arme nach innen, sodass die Nadel mit dem Nadelschutzelement 12 an der Nadelspitze aus dem Katheteransatz 1 herausgezogen werden kann.

Bei einer anderen Ausführungsform kann das Nadelschutzelement 12 mit dem Ventilbetätigungselement 9 bzw. mit dessen Führungsschenkeln 9b so in Eingriff stehen, dass das Nadelschutzelement 12 durch das Ventilbetätigungselement 9 gehalten wird, bis das Nadelschutzelement durch Aufnahme der Nadelspitze in die Schutzstellung gelangt, worauf die Klemmverbindung mit dem Ventilbetätigungselement 9 gelöst wird und das Nadelschutzelement 12 mit der Nadel 7 aus dem Katheteransatz 1 herausgezogen werden kann.

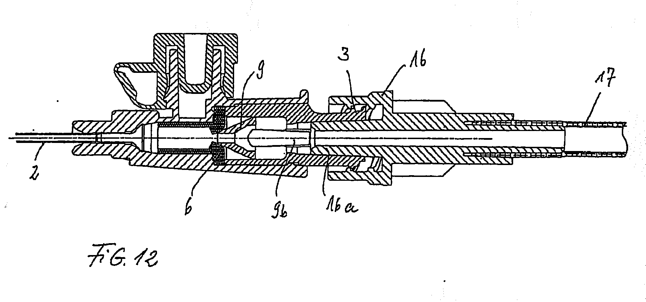

Fig. 12 zeigt ein Anschlusselement 16, das bei der Ausführungsform nach Fig. 10 auf dem Luer-Nocken 3 aufgeschraubt ist, wobei der in distale Richtung vorspringende, rohrförmige Ansatz 16a das Ventilbetätigungselement 9 über die Führungsschenkel 9b so beaufschlagt, dass die Ventilscheibe 6 geöffnet ist. In dieser in Fig. 12 wiedergegebenen Stellung kann über eine an das Anschlussstück 16 angeschlossene Leitung 17 Blut entnommen werden.

In Fig. 11 wird das Ventilbetätigungselement 9 durch den Nadelansatz 7b nicht beaufschlagt, sodass das Ventilbetätitungselement 9 in der in Fig. 9 wiedergegebenen Stellung verbleibt. Lediglich die Nadel 7 wird durch die Ventilscheibe 6 geführt wobei die zwischen den Ventilschlitzen 6a liegenden elastischen Bereiche der z. B. aus Silicon bestehenden Ventilscheibe 6 auf dem Außenumfang der Nadel 7 anliegen und Blut von der Nadel abstreifen, wenn die Nadel 7 aus dem Katheteransatz 1 zurückgezogen wird.

Das Ventilbetätigungselement 9 kann auch mit einer Abstreifeinrichtung versehen sein, wie in DE 20 2006 017 732 beschrieben, beispielsweise in Form eines die Nadel umgebenden Abstreifrings in dem Durchlass 9c (Fig. 3) des Ventilbetätigungselementes 9.

Fig. 8a zeigt einen Abstreifer 90 aus einem flüssigkeitsundurchlässigen Film, wie z.B. einen Polyethylenfilm, der eine im Wesentlichen kreisförmige Gestalt mit zwei Ausschnitten 91 umfasst. Die Ausschnitte 91 sind so dimensioniert und geformt, dass sie an dem Ventilbetätigungselement 9 angebracht werden können und die Führungsschenkel 9b aufnehmen. In einem Ausfiihrungsbeispiel weist der Abstreifer 90 einen durchgehend mittigen Abschnitt (d.h., keine mittige Öffnung) auf, die zum Durchbohren durch eine Nadel während des Zusammenbaus ausgebildet ist. Wenn die Nadel 7 aus dem Katheteransatz 1 zurückgezogen wird, wird Blut vom Nadelumfang durch den Abstreifer 90 abgestreift.

Bei einer solchen Ausgestaltung mit einem Abstreifer 90 kann bei Blutentnahme auf der proximalen Seite des Katheteransatzes das Blut um das Ventilbetätigungselement mit Abstreifer fließen, wobei Blut auch durch das Loch im Abstreifer 90 austreten kann, das durch die Nadel eingestochen wurde. Es können auch Nuten in Achsrichtung auf dem kegelstumpfförmigen Kopfteil 9a ausgebildet sein, um die Blutentnahme zu begünstigen.

Fig. 8b zeigt eine perspektivische Ansicht eines Ventilbetätigungselementes 9 mit einem kegelstumpfförmigen Kopfteil 9a und zwei Führungsschenkeln 9b, die mit dem Kopfteil verbunden sind und sich proximal von diesem erstrecken. Der Durchlass am Kopfteil weist eine trichterförmige Gestalt auf. In der Darstellung erstrecken sich die zwei Schenkel 9b parallel und versetzt in Bezug auf eine Achse, die durch die Mitte des Durchlasses bestimmt wird; sie können jedoch auch radial nach außen abgewinkelt sein, wobei sie sich proximal erstrecken. Die zwei Schenkel können auch einen leichten Bogen oder eine leichte Krümmung aufweisen.

Fig. 7 zeigt das in Fig. 11 in einer Seitenansicht wiedergegebene Nadelschutzelement 12 mit sich kreuzenden Armen in einer Draufsicht. Anstelle eines solchen Nadelschutzelementes 12 mit sich kreuzenden Armen kann auch eine andere Ausführungsform eines Nadelschutzelements vorgesehen werden, durch das beim Zurückziehen der Nadel die Nadelspitze abgedeckt und blockiert wird, sodass keine Verletzung durch die Nadelspitze möglich ist.

### Weitere Ausführungsformen der vorliegenden Erfindung

In einer Ausführungsform betrifft die Erfindung eine Kathetervorrichtung umfassend einen hohlen Katheteransatz 1, dessen distales Ende mit einem Katheter 2 verbindbar ist und an dessen proximalem Ende eine Verbindungseinrichtung 3 für eine zusätzliche Einrichtung vorgesehen ist, einen zwischen proximalem und distalem Ende radial abzweigenden Port 4 am Katheteransatz 1, ein erstes Ventilelement 5 in dem Katheteransatz 1, das in der Bereitstellung den Port 4 verschließt und bei Anliegen von Druck von außen am Port die Verbindung zwischen Port und Katheter freigibt, und ein zweites Ventilelement 6, das das Austreten von Blut aus dem Katheteransatz in proximale Richtung verhindert, dadurch gekennzeichnet, dass das zweite Ventilelement als Zwei-Wege-Ventil derart ausgebildet ist, dass eine Durchströmung in beiden Richtungen möglich ist.

In einer bevorzugten Ausführungsform ist das erste Ventilelement 5 in Form eines flexiblen Schlauchelementes ausgebildet, das in der Bereitstellung auf dem Innenumfang des Katheteransatzes 1 anliegt und bei Anliegen von Druck von außen am Port die Verbindung zwischen Port und Katheter durch Deformation freigibt.

In einem weiteren Ausführungsbeispiel ist das zweite Ventilelement durch eine das proximale Ende des ersten Ventilelements 5 verschließende Ventilscheibe 6 ausgebildet, die mit wenigstens einem radial verlaufenden Schlitz 6a versehen ist.

Bei einer weiteren Ausführungsform ist am proximalen Ende des Schlauchelementes 5 ein radial abstehender Flansch 5a ausgebildet, der in eine Ringnut auf dem Innenumfang des Katheteransatzes 1 eingreift.

In einer weiteren Ausführungsform ist am proximalen Ende des Schlauchelementes 5 auf dessen Innenumfang ein Versteifungsring 5b eingesetzt.

In einem weiteren Ausführungsbeispiel ist auf der proximalen Seite des zweiten Ventilelementes 6 ein Ventilbetätigungselement 9 im Katheteransatz 1 in Achsrichtung verschiebbar geführt.

Bei einer weiteren Ausführungsform ist das Ventilbetätigungselement 9 durch ein Anschlusselement 11, 16 in distale Richtung im Katheteransatz 1 zum Öffnen des zweiten Ventilelementes 6 verschiebbar.

In einer weiteren Ausführungsform ist das zweite Ventilelement 6 derart ausgebildet, dass es selbsttätig schließt, wenn das Ventilbetätigungselement 9 auf das Ventilelement 6 keine Kraft mehr ausübt.

In einer weiteren Ausführungsform ist auf der proximalen Seite des zweiten Ventilelementes 6 ein Nadelschutzelement 12 auf einer in die Kathetervorrichtung eingesetzten Nadel 7 verschiebbar geführt.

Bei einer weiteren Ausführungsform ist auf dem Innenumfang des Katheteransatzes 1 eine Halteeinrichtung 1d für das Nadelschutzelement 12 ausgebildet.

In einem weiteren Ausführungsbeispiel ist das Nadelschutzelement 12 mit wenigstens einem radial abstehenden Arm 130 versehen, mittels dem das Nadelschutzelement im Katheteransatz durch Klemmen gehalten ist.

In einem weiteren Ausführungsbeispiel ist das erste Ventilelement bildende Schlauchelement 5 im Bereich der Portöffnung 4a mit einem in Achsrichtung verlaufenden Schlitz 5c versehen.

## Patentansprüche

1. Kathetervorrichtung, umfassend
einen Katheter (2),
einen hohlen Katheteransatz (1), dessen distales Ende mit dem Katheter (2) verbunden ist und an dessen proximalem Ende eine Verbindungseinrichtung (3) für eine zusätzliche Einrichtung vorgesehen ist,
einen zwischen proximalem und distalem Ende radial abzweigenden Port (4) am Katheteransatz (1),
ein Ventilelement (6), das das Austreten von Blut aus dem Katheteransatz (1) in proximale Richtung verhindert, wobei das Ventilelement (6) als Zwei-Wege-Ventil derart in dem Katheteransatz (1) ausgebildet ist, dass eine Durchströmung in beiden Richtungen möglich ist, wobei Flüssigkeit oder Blut am proximalen Ende des Katheteransatzes (1) entnommen werden kann,
wobei das Ventilelement (6) eine mit wenigstens einem radial verlaufenden Schlitz (6a) versehene Ventilscheibe ist,
wobei auf der proximalen Seite des Ventilelementes (6) ein Ventilbetätigungselement (9) vollständig im Katheteransatz (1) angeordnet und in Achsrichtung verschiebbar geführt ist, und wobei das Ventilbetätigungselement (9) das Ventilelement (6) durch Verschiebung in distale Richtung für eine Durchströmung in beiden Richtungen öffnet, **dadurch gekennzeichnet,**
**dass** ein weiteres Ventilelement (5) in dem Katheteransatz (1) vorhanden ist, das in der Bereitstellung den Port (4) verschließt, wobei das Ventilelement (6) proximal von dem weiteren Ventilelement (5) angeordnet ist.

2. Kathetervorrichtung nach Anspruch 1, wobei das Ventilbetätigungselement (9) durch ein Anschlusselement (11, 16) in distale Richtung im Katheteransatz (1) zum Öffnen des Ventilelementes (6) verschiebbar ist.

3. Kathetervorrichtung nach Anspruch 1 oder 2, wobei das Ventilbetätigungselement (9) einen Kopfteil (9a) aufweist.

4. Kathetervorrichtung nach Anspruch 3, wobei der Kopfteil (9a) mit einer Durchgangsbohrung (9c) versehen ist und der Kopfteil (9a) derart ausgebildet ist, dass das Ventilbetätigungselement (9) in einem der radial verlaufenden Schlitze des Ventilelements (6) einrastet.

5. Kathetervorrichtung nach einem der Ansprüche 3 oder 4, wobei das Ventilbetätigungselement (9) zwei Führungsschenkel (9b) aufweist, die mit dem Kopfteil (9a) verbunden sind und von dem Kopfteil (9a) proximal in Achsrichtung abstehen.

6. Kathetervorrichtung nach einem der Ansprüche 3 bis 5, wobei der Kopfteil (9a) mit einer Abstufung (9d) zum Einrasten des Ventilbetätigungselements (9) in einem der radial verlaufenden Schlitze des Ventilelements (6) versehen ist.

7. Kathetervorrichtung nach einem der vorhergehenden Ansprüche, wobei das weitere Ventilelement (5) bei Anliegen von Druck am Port (4) eine Verbindung zwischen dem Port (4) und dem Katheter (2) freigibt.

8. Kathetervorrichtung nach einem der vorhergehenden Ansprüche, wobei das weitere Ventilelement (5) mit einem in Achsrichtung verlaufenden Schlitz (5c) versehen ist.

9. Kathetervorrichtung nach einem der vorhergehenden Ansprüche, wobei das weitere Ventilelement (5) mit einen Versteifungsring (5b) versehen ist.

10. Kathetervorrichtung nach Anspruch 9, wobei der Versteifungsring (5b) in einem Abstand vom proximalen Ende des Ventilelements (5) eingesetzt ist.

11. Kathetervorrichtung nach einem der vorhergehenden Ansprüche, wobei das weitere Ventilelement (5) in Form eines flexiblen Schlauchelements ausgestaltet ist, das sowohl ein Ventilelement (5) an der Portöffnung (4a) als auch ein Ventilelement im proximalen Bereich des Katheteransatzes (1) bildet.

12. Kathetervorrichtung nach einem der Ansprüche 1 bis 10, wobei das Ventilelement (5) und Ventilelement (6) voneinander getrennte Ventilelemente sind.

13. Kathetervorrichtung nach einem der vorhergehenden Ansprüche, wobei auf der proximalen Seite des Ventilelementes (6) ein Nadelschutzelement (12) auf einer in die Kathetervorrichtung eingesetzten Nadel (7) verschiebbar geführt ist.

14. Kathetervorrichtung nach Anspruch 13, wobei das Nadelschutzelement (12) mit einer proximalen Rückwand versehen ist.

15. Kathetervorrichtung nach Anspruch 14, wobei die Nadel (7) mit einer Nadelquetschung versehen ist, die beim Zurückziehen der Nadel (7) mit der proximalen Rückwand des Nadelschutzelements (12) in Eingriff tritt.

16. Kathetervorrichtung nach einem der Ansprüche 13 bis 15, wobei die Nadelspitze in zurückgezogener Position durch das Nadelschutzelement (12) blockiert ist.

17. Kathetervorrichtung nach einem der Ansprüche 13 bis 16, weiterhin umfassend eine Halteeinrichtung (1d) für das Nadelschutzelement (12).

18. Kathetervorrichtung nach Anspruch 17, wobei die Halteeinrichtung (1d) auf dem Innenumfang des Katheteransatzes (1) ausgebildet ist.

19. Kathetervorrichtung nach einem der vorhergehenden Ansprüche, weiterhin umfassend einen Abstreifer (90).

20. Kathetervorrichtung nach Anspruch 19, wobei der Abstreifer (90) einen durchgehend mittigen Abschnitt ohne mittige Öffnung zum Durchbohren der Nadel während des Zusammenbaus aufweist.

21. Kathetervorrichtung nach einem der vorhergehenden Ansprüche, wobei das proximale Ende des Katheteransatzes (1) eine Verschlusskappe (15a) umfasst, mit der das proximale Ende des Katheteransatzes (1) verschlossen gehalten werden kann, wenn über den Port (4) eine Injektion ausgeführt wird.

## Claims

1. A catheter device comprising
a catheter (2),
a hollow catheter hub (1), the distal end of which is connected to the catheter (2) and at the proximal end of which a connection means (3) for an additional device is provided,
a radially branching port (4) between the proximal end and the distal end on the catheter hub (1),
a valve element (6), which prevents blood from emerging from the catheter hub (1) in a proximal direction, wherein the valve element (6) is formed as a two-way-valve in the catheter hub (1), in such a way that throughflow is possible in both directions, wherein liquid or blood can be sampled at the proximal end of the catheter hub (1),
wherein the valve element (6) is a valve disc provided with at least one radially extending slit (6a),
wherein on the proximal side of the valve element (6) a valve actuation element (9) is completely arranged in the catheter hub (1) and guided displaceably in an axial direction, and wherein the valve actuation element (9) opens the valve element (6) by displacement in the distal direction in order to provide a throughflow in both directions,
**characterized in that**
a further valve element (5) is provided in the catheter hub (1), which closes the port (4) in the ready position, wherein the valve element (6) is positioned proximal of the further valve element (5).

2. The catheter device of claim 1, wherein the valve actuation element (9) is displaceable in the distal direction in the catheter hub (1) by means of a connection element (11, 16) for opening the valve element (6).

3. The catheter device of claim 1 or 2, wherein the valve actuation element (9) has a head part (9a).

4. The catheter device of claim 3, wherein the head part (9a) is provided with a passage bore (9c), and the head part (9a) is formed in such a way that the valve actuation element (9) latches into one of the radially extending slits of the valve element (6).

5. The catheter device of any of claims 3 or 4, wherein the valve actuation element (9) comprises two guide limbs (9b), which are connected to the head part (9a) and which protrude proximally in the axial direction from the head part (9a).

6. The catheter device of any of claims 3 to 5, wherein the head part (9a) is provided with a step (9d) for latching the valve actuation element (9) in one of the radially extending slits of the valve element (6).

7. The catheter device of any of the preceding claims, wherein the further valve element (5) releases a connection between the port (4) and the catheter (2) when pressure is applied on the port (4).

8. The catheter device of any of the preceding claims, wherein the further valve element (5) is provided with a slit (5c) extending in the axial direction.

9. The catheter device of any of the preceding claims, wherein the further valve element (5) is provided with a reinforcing ring (5b).

10. The catheter device of claim 9, wherein the reinforcing ring (5b) is inserted at a distance from the proximal end of the valve element (5).

11. The catheter device of any of the preceding claims, wherein the further valve element (5) is configured as a flexible hose element, which forms both a valve element (5) at the port opening (4a) and a valve element at the proximal region of the catheter hub (1).

12. The catheter device of any of claims 1 to 10, wherein the valve element (5) and the valve element (6) are separate valve elements.

13. The catheter device of any of the preceding claims, wherein, on the proximal side of the valve element (6), a needle protection element (12) is displaceably guided on a needle (7) inserted into the catheter device.

14. The catheter device of claim 13, wherein the needle protective element (12) is provided with a proximal rear wall.

15. The catheter device of claim 14, wherein the needle (7) is provided with a needle crimp, which, when the needle (8) is retracted, engages the proximal rear wall of the needle protective element (12).

16. The catheter device of any of claims 13 to 15, wherein the needle tip is blocked by the needle protective element (12) in the retracted position.

17. The catheter device of any of claims 13 to 16, further comprising a holding means (1d) for the needle protective element (12).

18. The catheter device of claim 17, wherein the holding means (1d) is formed on the inner circumference of the catheter hub (1).

19. The catheter device of any of the preceding claims, further comprising a wiper (90).

20. The catheter device of claim 19, wherein the wiper (90) has a continuous central portion without a central opening for boring through the needle during assembly.

21. The catheter device of any of the preceding claims, wherein the proximal end of the catheter hub (1) comprises a sealing cap (15a) with which the proximal end of the catheter hub (1) can be kept sealed, when an injection is carried out via the port (4).

## Revendications

1. Dispositif de cathéter, comprenant
un cathéter (2)
un embout de cathéter creux (1), dont l'extrémité distale est reliée au cathéter (2) et dans lequel une installation de raccordement (3) pour une installation supplémentaire est prévue au niveau de l'extrémité proximale,
un port déviant de manière radiale (4) entre l'extrémité proximale et distale au niveau de l'embout de cathéter (1),
un élément de soupape (6) qui empêche la fuite de sang en dehors de l'embout de cathéter dans la direction proximale, l'élément de soupape (6) étant conçu, dans l'embout de cathéter, comme une soupape à deux voies, de sorte qu'une circulation est possible dans les deux sens, le liquide ou le sang pouvant être éliminé au niveau de l'extrémité proximale de l'embout de cathéter (1),
l'élément de soupape (6) étant un disque de soupape pourvu d'au moins une fente s'étendant de manière radiale (6a),
un élément d'actionnement de soupape (9) étant disposé entièrement dans l'embout de cathéter (1) sur le côté proximal de l'élément de soupape (6) et dirigé de manière coulissante dans la direction axiale, et l'élément d'actionnement de soupape (9) ouvrant l'élément de soupape (6) par coulissage dans la direction distale pour une circulation dans les deux sens,
**caractérisé en ce que**
un autre élément de soupape (5) est disponible dans l'embout de cathéter (1), lequel élément de soupape ferme le port (4) lors de la mise à disposition, l'élément de soupape (6) étant disposé de manière proximale par rapport à l'autre élément de soupape (5).

2. Dispositif de cathéter selon la revendication 1, dans lequel l'élément d'actionnement de soupape (9) peut être coulissé à travers un élément de raccordement (11, 16) dans la direction distale dans l'embout de cathéter (1) pour l'ouverture de l'élément de soupape (6).

3. Dispositif de cathéter selon la revendication 1 ou 2, dans lequel l'élément d'actionnement de soupape (9) présente une partie de tête (9a).

4. Dispositif de cathéter selon la revendication 3, dans lequel la partie de tête (9a) est pourvu d'un alésage traversant (9c) et la partie de tête (9a) est conçue de sorte que l'élément d'actionnement de soupape (9) s'emboîte dans l'une des fentes s'étendant de manière radiale de l'élément de soupape (6).

5. Dispositif de cathéter selon l'une des revendications 3 ou 4, dans lequel l'élément d'actionnement de soupape (9) présente deux branches de guidage (9b) raccordées à la partie de tête (9a) et faisant saillie à partir de la partie de tête (9a) de manière proximale dans la direction axiale.

6. Dispositif de cathéter selon l'une des revendications 3 à 5, dans lequel la partie de tête (9a) est pourvue d'une gradation (9d) pour l'emboîtement de l'élément d'actionnement de soupape (9) dans l'une des fentes s'étendant de manière radiale de l'élément de soupape (6).

7. Dispositif de cathéter selon l'une des revendications précédentes, dans lequel l'autre élément de soupape (5) libère un raccordement entre le port (4) et le cathéter (2) lors de l'application d'une pression sur le port (4).

8. Dispositif de cathéter selon l'une des revendications précédentes, dans lequel l'autre élément de soupape (5) est pourvu d'une fente (5c) s'étendant dans la direction axiale.

9. Dispositif de cathéter selon l'une des revendications précédentes, dans lequel l'autre élément de soupape (5) est pourvu d'une bague de renforcement (5b).

10. Dispositif de cathéter selon la revendication 9, dans lequel la bague de renforcement (5b) est insérée dans un espacement de l'extrémité proximale de l'élément de soupape (5).

11. Dispositif de cathéter selon l'une des revendications précédentes, dans lequel l'autre élément de soupape (5) est agencé sous forme d'un élément de tube souple qui forme un élément de soupape (5) au niveau de l'ouverture de port (4a) ainsi qu'un élément de soupape dans la zone proximale de l'embout de cathéter (1).

12. Dispositif de cathéter selon l'une des revendications 1 à 10, dans lequel l'élément de soupape (5) et l'élément de soupape (6) sont des éléments de soupape séparés l'un de l'autre.

13. Dispositif de cathéter selon l'une des revendications précédentes, dans lequel un élément de protection d'aiguille (12) est dirigé par coulissage sur l'une des aiguilles (7) insérées dans le dispositif de cathéter sur le côté proximal de l'élément de soupape (6).

14. Dispositif de cathéter selon la revendication 13, dans lequel l'élément de protection d'aiguille (12) est pourvu d'une paroi arrière proximale.

15. Dispositif de cathéter selon la revendication 14, dans lequel l'aiguille (7) est pourvue d'une contusion d'aiguille qui vient en prise avec la paroi arrière proximale de l'élément de protection d'aiguille (12) lors du retrait de l'aiguille (7).

16. Dispositif de cathéter selon l'une des revendications 13 à 15, dans lequel la pointe d'aiguille est bloquée en position retirée par l'élément de protection d'aiguille (12).

17. Dispositif de cathéter selon l'une des revendications 13 à 16, comprenant en outre un dispositif d'arrêt (1d) pour l'élément de protection d'aiguille (12).

18. Dispositif de cathéter selon la revendication 17, dans lequel le dispositif d'arrêt (1d) est formé sur le pourtour intérieur de l'embout de cathéter (1).

19. Dispositif de cathéter selon l'une des revendications précédentes, comprenant en outre un racleur (90).

20. Dispositif de cathéter selon la revendication 19, dans lequel le racleur (90) présente une section centrée de manière continue sans ouverture centrée pour l'alésage de l'aiguille lors du montage.

21. Dispositif de cathéter selon l'une des revendications précédentes, dans lequel l'extrémité proximale de l'embout de cathéter (1) comprend un capuchon de fermeture (15a) avec lequel l'extrémité proximale de l'embout de cathéter (1) peut être maintenue fermée si une injection est exécutée par le port (4).
